(19) Europäisches Patentamt
European Patent Office
Office européen des brevets

(11) **EP 3 925 531 A1**

(12) **EUROPEAN PATENT APPLICATION**

(43) Date of publication:
**22.12.2021 Bulletin 2021/51**

(21) Application number: **20180998.5**

(22) Date of filing: **19.06.2020**

(51) Int Cl.:
*A61B 5/08* (2006.01)        *A61B 5/09* (2006.01)
*A61B 5/097* (2006.01)       *A61B 5/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• ZHANG, Xuan
  **5656 AE Eindhoven (NL)**
• SU, Wei
  **5656 AE Eindhoven (NL)**

(74) Representative: **Philips Intellectual Property & Standards**
**High Tech Campus 52**
**5656 AG Eindhoven (NL)**

(54) **POSTURE MONITORING SYSTEM AND METHOD**

(57)    A posture monitoring system comprises a mask having a fan for drawing air from outside a mask air chamber into the air chamber and/or drawing air from inside the air chamber to the outside. A rotation speed of the fan is monitored, and from this monitoring, a first value is derived which relates to a depth of breathing and a second value is derived which relates to a rate of breathing. Information relating to the posture of the user is derived from the first and second values.

FIG. 3

## Description

FIELD OF THE INVENTION

This invention relates to posture monitoring systems and methods.

BACKGROUND OF THE INVENTION

Sustained poor posture is associated with the development and worsening of many musculoskeletal disorders. Especially for children, poor spinal posture can not only affect their health in the present (e.g. back pain) but also have long term effects on their body and render their spine vulnerable to injuries, because their skeleton is not yet mature and is experiencing significant growth.

Unfortunately, in recent years, the weight of a school backpack has become a growing concern, not just among parents but also among healthcare professionals and school administrations, since school children have been increasingly complaining of backaches. Approximately 12% of the world population is constituted by children carrying backpacks to school every day.

Studies show that with increasing school loads, these children are carrying backpacks heavier than 10% of their body weight on a regular basis. Regardless of whether a child carries the backpack over one shoulder or two, backaches have been widely reported by the children.

Carrying a backpack which is too heavy will cause health issues to the children. When carrying a heavy backpack, the posture is changed, with the child bending their spine forward to maintain body posture and balance during walking. This unnatural posture will influence their spinal development as time passes.

It is not realistic to prohibit the carrying of schoolbags. It would therefore be of interest to provide real-time spinal posture monitoring, for example to guide a person to a better posture to improve their spinal shape, with consequent general health and wellbeing benefits.

There are commercially available devices with a spine posture monitoring function, such as a patch for application against the spine (by straps or integrated into a garment, for example). However, it is not convenient to wear such a device often in daily life.

There is therefore a need for a more convenient way to monitor posture.

SUMMARY OF THE INVENTION

The invention is defined by the claims.

According to examples in accordance with an aspect of the invention, there is provided a posture monitoring system, comprising:

a mask which defines an air chamber over the nose and mouth of a user;
a fan for rotating at a rotation speed and drawing air from outside the air chamber into the air chamber and/or drawing air from inside the air chamber to the outside; and
a controller configured to:

determine from the rotation speed of the fan a first value which relates to a depth of breathing and a second value which relates to a rate of breathing; and
determine from the first and second values information relating to the posture of the user.

This system makes use of a simple fan-assisted mask to monitor breathing characteristics of the wearer, and from this derive information relating to the posture of the wearer. This posture information for example may simply indicate whether or not a correct posture is being used. It may however provide additional information such as the type of issue with the posture or how to correct the posture. By using monitoring of the fan rotation speed, a simple system is provided with few sensors. Indeed, the sensors required may already be present for the other functions of the mask, such as the fan speed control. The mask is for example a pollution mask.

A pollution mask is a device which has the primary purpose of filtering ambient air to be breathed by the user. The mask does not perform any form of patient treatment. In particular, the pressure levels and flows resulting from the fan operation are intended solely to assist in providing comfort (by influencing the temperature or relative humidity in the air chamber) and/or to assist in providing a flow across a filter without requiring significant additional breathing effort by the user. The mask does not provide overall breathing assistance compared to a condition in which the user does not wear the mask. Thus, it is an everyday mask used when the wearer is in perfect health, to provide protection from pollutants.

The rotation speed of the fan may be known to the controller based on electrical control signals of the fan, or based on rotor position signals which exist as part of the internal control mechanism of the fan motor, or based on sensing the rotation of the fan using a sensor. These different approaches may each be considered to constitute a fan rotation speed monitoring system.

The first value is for example a tidal volume. Studies show that when children bend their spine forward, the poor spinal posture will constrain the diaphragmatic breathing, since the outward movement of the abdomen will be restricted by abdominal muscular contraction to stabilize the lower trunk and reduce the upper trunk range of motion. The result is that more rapid costal breathing giving an increase in breathing frequency but not tidal volume.

The controller for example also controls the fan speed. It may for example control the fan in synchronism with the breathing cycles of the user, in order to save power. For the example of an exhalation fan, it may turn off during inhalation. For the example of an inhalation fan, it may turn off during exhalation.

The system for example comprises a filter which forms a boundary between the air chamber and the ambient surroundings outside the air chamber. This is a standard part of a pollution mask.

The controller is then configured to obtain the tidal volume by:

determining a pressure between the air chamber and the ambient surroundings;
analyzing the rotation speed of the fan over time and the pressure over time thereby to determine breathing flow rate information, taking into account the permeability characteristics of the filter.

The controller is used to identify breathing cycles (i.e. inhalation and exhalation cycles) as well as flow rates. The breathing flow information is derived without a direct flow measurement. It is instead derived from pressure values (and known characteristics of the structure of the mask).

The controller is for example configured to derive the pressure between the air chamber and the ambient surroundings from the rotation speed of the fan, such that the fan speed is used as a proxy of pressure measurement.

In this way, the fan speed (for a fan which drives air into the chamber and/or expels it from the chamber) is used as a proxy of pressure measurement. To measure the fan speed, the circuitry of the fan itself may be used so that no additional sensors are required. The chamber may be closed in normal use, so that pressure fluctuations in the chamber have an influence on the load conditions of the fan and hence alter the fan electrical characteristics. This avoids the need for a separate pressure sensor.

The controller may be configured to:

derive a filter flow rate through the filter based on the pressure and on the permeability characteristics of the filter;
derive a fan flow rate from the fan rotation speed; and
derive a breathing flow rate based on a sum of, or difference between, the filter flow rate and the fan flow rate.

The permeability characteristics may be known in advance and taken into account in the algorithm implemented by the controller. For example, filter permeability information can be calibrated at the production line. For instance, after filter manufacture, the filter permeability can be measured by a flow rate and pressure measurement device. Permeability information can then be written into a memory of the controller for use by the algorithm run by the controller.

By combining information about the fan flow rate (from the fan speed) and the filter flow rate (from the pressure difference across the filter), the breathing flow from the user, e.g. through the nose, can be determined.

The controller is preferably configured to determine the timing of inhaling and exhaling from the pressure (e.g. proxy pressure), and to derive the breathing flow rate during inhalation or exhalation. A tidal volume can then be derived from the breathing flow rate over the time of a breathing inhalation or exhalation.

The first value is for example based on a maximum swing in fan rotation speed during a sampling window and the second value is a frequency based on the time between a consecutive maxima and minima in the fan rotation speed.

The controller may be part of a system remote to the mask, with wireless communication between the controller and the mask. The controller may for example be part of a mobile (smart) phone on which an app is loaded.

The controller is preferably configured to implement a calibration routine, and thereby determine an expected relationship between each of the first and second values and an exercise intensity when the user is carrying no additional load. This enables a new user to record their breathing characteristics. These characteristics can then be compared with a general database to validate baseline readings for the user.

The controller is then configured to implement a monitoring function after the calibration routine, and thereby determine when the first and/or second values depart from the expected relationships with the exercise intensity, and thereby determine that the user has an incorrect posture.

The invention also provides a posture monitoring method, comprising:

using a controller to control a fan of a mask which defines an air chamber over the nose and mouth of a user, wherein the fan is for drawing air from outside the air chamber into the air chamber and/or drawing air from inside the air chamber to the outside;

monitoring a rotation speed of the fan;

determining from the rotation speed of the fan a first value which relates to a depth of breathing and a second value which relates to a rate of breathing; and

determining from the first and second values information relating to the posture of the user.

Determining a first value may comprise determining a tidal volume, by:

determining a pressure between the air chamber and the ambient surroundings from the rotation speed of the fan, such that the fan speed is used as a proxy of pressure measurement; and

analyzing the rotation speed of the fan over time and the pressure over time thereby to determine breathing flow rate information, taking into account the permeability characteristics of the filter.

The method may comprise:

implementing a calibration routine, and thereby determine an expected relationship between the each of the first and second values and an exercise intensity when the user is carrying no additional load; and

implementing a monitoring function after the calibration routine, and thereby determine when the first and/or second values depart from the expected relationship with the exercise intensity, and thereby determine that the user has an incorrect posture.

The method of the invention may be implemented by a computer program.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows the relationship between respiratory rate (/minute, y-axis) and exercise rate (W, x-axis);

Figure 2 shows the relationship between tidal volume (L, y-axis) and exercise rate (W, x-axis);

Figure 3 shows a combination of the two plots of Figures 1 and 2;

Figure 4 shows a face mask for implementing the approach of the invention;

Figure 5 shows one example of the components of the system;

Figure 6A shows schematically the rotor position (as a measured sensor voltage) against time of the fan motor;

Figure 6B shows the frequency variation over time of the fan motor;

Figure 7 shows an H-bridge circuit which functions as an inverter to generate an alternating voltage;

Figure 8A shows the air flow situation when the user is breathing out and with air flow entering the mask volume;

Figure 8B shows the air flow situation when the user is breathing out and with air flow leaving the mask volume; and

Figure 9 shows a posture monitoring method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a posture monitoring system comprises a mask having a fan for drawing air from outside a mask air chamber into the air chamber and/or drawing air from inside the air chamber to the outside. A rotation speed of the fan is monitored, and from this monitoring, a first value is derived which relates to a depth of breathing and a second value is derived which relates to a rate of breathing. Information relating to the posture of the user is derived from the first and second values.

The invention thus integrates posture monitoring into a pollution mask. Pollution masks are increasingly common. Indeed, the World Health Organization (WHO) estimates that 4 million people die from air pollution every year. Part of this problem is the outdoor air quality in cities. The worst in class are Indian cities like Delhi that have an annual pollution level more than 10 times the recommended level. Well known is Beijing with an annual average 8.5 times the recommended safe levels. However, even in European cities like London, Paris and Berlin, the levels are higher than recommended by the WHO.

Since this problem will not improve significantly on a short time scale, the only way to deal with this problem is to wear a mask which provides cleaner air by filtration. To improve comfort and effectiveness one or two fans can be added to the mask. These fans are switched on during use and are typically used at a constant voltage. For efficiency and longevity reasons these are normally electrically commutated brushless DC fans.

The benefit to the wearer of using a powered mask is that the lungs are relieved of the slight strain caused by inhalation and/or exhalation against the resistance of the filters in a conventional non-powered mask.

There are several advantages if the fan operation or speed is regulated. This can be used to improve comfort by more appropriate ventilation during the inhalation and exhalation sequence or it can be used to improve the electrical efficiency. The latter translates into longer battery life or increased ventilation. Both of these aspects need improvement in current designs.

WO 2018/215225 discloses a mask in which a rotation speed of the fan is used as a proxy for pressure measurement. A pressure or a pressure change is determined based on the rotation speed of the fan. Using this pressure information, the breathing pattern of the user can be tracked.

This invention is based on the use of the breathing pattern information to derive information about the posture of the wearer. Children are particularly vulnerable to the effects of air pollution for several biological reasons. Children's lungs, immune system, and brain are immature and continue to develop rapidly, and the cell layer lining the inside of the respiratory tract is particularly permeable. Children breathe more air per kilogram of body mass than adults. Air pollution exposure in childhood has a high risk of creating lasting effects on future health.

Therefore, wearing pollution masks is more important for children when outside, including the daily commute to school or college.

The invention is based on the use of fan rotation signals of an active mask as to measure the users' breathing pattern (as already proposed in WO 2018/215225) and further to monitor derive from this breathing pattern spinal posture information, for example to provide feedback when poor posture occurs. Long-term usage of such a mask may instill correct postural habits and yield a decrease in the incidence of posture-related musculoskeletal disorders.

The way breathing characteristics may be used to derive posture information will now be explained.

Breathing frequency (breaths per minute) and tidal volume (volume of air moved into or out of the lungs during one breathing cycle) are two key respiratory parameters. Generally, with the increase of activity intensity, both breathing frequency and tidal volume will increase to meet the requirement of increased metabolic cost.

However, studies show that when children bend their spine forward due to a heavy backpack, the poor spinal posture will constrain the diaphragmatic breathing, since the outward movement of the abdomen will be restricted by abdominal muscular contraction to stabilize the lower trunk and reduce the upper trunk range of motion. Thus, the only way that the subjects could increase oxygen uptake to support the increased metabolic cost might be to use costal breathing and breathe faster.

This has been reported in Jing Xian Li, Youlian Hong, Paul D. Robinson, "The effect of load carriage of movement kinematics and respiratory parameters in children during walking", Eur J Appl Physiol, 90, 35-43, 2003. The increased metabolic cost caused by carrying a too heavy backpack will mainly result in an increase in breathing frequency instead of both breathing frequency and tidal volume.

Therefore, the basic principle of the invention is to monitor the spinal posture by detecting the effect of poor posture on the change of the respiratory pattern.

The system for example makes use of a database in which breathing pattern data (including breathing frequency and tidal volume) is recorded for children with different ages, body weights and gender, and under different activity intensity (walking speeds). This data may be collected from various sources (e.g. literature studies, reports, real person tests, etc.) and stored in the cloud.

An example of data collected from a literature study is shown in Table 1, from Kristin S. Ondrak, Robert G. McMurray, "Exercise-induced breathing patterns of youth are related to age and intensity", Eur J Appl Physiol, 98, 88-96, 2006. This database also could be continuously extended and updated with the increasing number of users. The table shows the tidal volume $V_T$ relative to the body mass and the breathing frequency $f_B$.

Table 1 - part 1

| Age (year) | Gender | Body Mass (kg) | At rest | | Slow walking (4.0 km/h) | |
|---|---|---|---|---|---|---|
| | | | $V_T$ kg$^{-1}$ (mL/kg) | $f_B$ (min$^1$) | $V_T$ kg$^{-1}$ (mL/kg) | $f_B$ (min$^1$) |
| 8 | F | 29.5±4.7 | 13.2±3.8 | 21.9±5.4 | 18.2±3.7 | 35.4±6.7 |
| | M | 28.9±5.1 | 11.0±2.6 | 24.3±4.3 | 15.3±2.9 | 39.5±7.0 |
| 9 | F | 31.0±4.1 | 10.9±4.2 | 22.8±7.6 | 15.5±4.7 | 32.6±6.0 |
| | M | 32.7±6.4 | 12.1±5.3 | 20.7±5.1 | 16.4±1.9 | 34.4±3.3 |
| 10 | F | 39.8±8.0 | 86±2.1 | 22.6±6.9 | 15.4±3.5 | 32.3±6.5 |
| | M | 36.1±7.7 | 11.5±4.6 | 20.3±5.3 | 14.9±2.8 | 36.1±6.8 |
| 11 | F | 44.6±12.8 | 9.4±2.2 | 20.1±3.3 | 14.0±2.4 | 31.8±5.8 |
| | M | 41.6±7.8 | 10.3±2.9 | 19.7±4.1 | 14.9±2.4 | 31.2±7.2 |
| 12 | F | 49.7±9.3 | 8.4±2.5 | 20.2±2.9 | 15.4±3.3 | 27.3±4.2 |
| | M | 49.8±12.2 | 9.7±3.2 | 22.8±12.5 | 15.0±3.4 | 32.0±6.4 |
| 13 | F | 58.1±13.4 | 8.8±3.9 | 17.9±3.2 | 14.3±3.6 | 26.9±5.2 |
| | M | 49.4±8.6 | 10.3±4.6 | 19.1±4.8 | 15.5±2.8 | 26.5±3.6 |
| 14 | F | 57.3±17.8 | 8.2±3 .3 | 21.7±3.3 | 14.0±3 .0 | 28.9±4.5 |
| | M | 57.2±15.2 | 8.4±2.5 | 19.7±4.1 | 14.1±3.1 | 28.6±7.0 |
| 15 | F | 67.8±15.9 | 6.7±1.5 | 19.9±2.3 | 14.1±2.4 | 26.8±3.3 |
| | M | 63.6±8.2 | 9.6±2.4 | 16.2±4.5 | 15.0±2.0 | 24.6±3.7 |
| 16 | F | 64.1±12.9 | 7.8±1.9 | 18.3±4.2 | 13.0±2.1 | 26.6±5.8 |
| | M | 69.6±10.8 | 9.4±2.5 | 16.4±3.2 | 16.4±5.6 | 22.4±5.9 |
| 17 | F | 60.7±9.3 | 7.8± 1.1 | 19.8±2.8 | 11.9±1.5 | 28.4±3.7 |
| | M | 72.4±11.0 | 7.5±1.5 | 17.1±3.2 | 13.9±2.5 | 23.3±4.1 |
| 18 | F | 61.3±8.3 | 7. 8±2.2 | 18.2±4.7 | 13.9±3.7 | 25.6±6.5 |
| | M | 68.7±12.1 | 8.3±2.7 | 17.0±3.8 | 13.2±2.2 | 25.3±4.4 |

Table 1 - part 2

| Age (year) | Gender | Body Mass (kg) | Fast walking (5.6 km/h) | | Running (8.0 km/h) | |
|---|---|---|---|---|---|---|
| | | | $V_T$ kg$^{-1}$ (mL/kg) | $f_B$ (min$^1$) | $V_T$ kg$^{-1}$ (mL/kg) | $f_B$ (min$^1$) |
| 8 | F | 29.5±4.7 | 20.0±5.1 | 45.2±7.1 | 21.3±2.9 | 64.8±8.6 |
| | M | 28.9±5.1 | 17.4±3.3 | 49.7±9.1 | 19.4±2.8 | 72.0±10.6 |
| 9 | F | 31.0±4.1 | 17.5±4.2 | 41.6±7.0 | 21.1±3.1 | 66.3±12.0 |
| | M | 32.7±6.4 | 20.8±4.7 | 39.1±3.1 | 23.3±5.0 | 63.0±12.0 |
| 10 | F | 39.8±8.0 | 18.2±4.1 | 39.9±9.3 | 24.2±3.5 | 51.7±6.9 |
| | M | 36.1±7.7 | 17.7±3.3 | 42.0±8.7 | 22.7±3.2 | 57.5±8.3 |
| 11 | F | 44.6±12.8 | 16.6±2.9 | 37.2±7.3 | 21.4±4.0 | 55.9±10.7 |
| | M | 41.6±7.8 | 16.7±2.8 | 40.6±11.3 | 21.6±2.5 | 59.2±7.8 |
| 12 | F | 49.7±9.3 | 17.5±2.9 | 32.2±4.9 | 22.7±4.9 | 52.2±10.4 |
| | M | 49.8±12.2 | 17.2±3.8 | 37.0±9.4 | 21.4±4.5 | 54.4±11.0 |
| 13 | F | 58.1±13.4 | 16.3±3.0 | 32.1±5.4 | 22.5±3.1 | 48.3±8.5 |

(continued)

| Age (year) | Gender | Body Mass (kg) | Fast walking (5.6 km/h) | | Running (8.0 km/h) | |
|---|---|---|---|---|---|---|
| | | | $V_T$ kg$^{-1}$ (mL/kg) | $f_B$ (min$^1$) | $V_T$ kg$^{-1}$ (mL/kg) | $f_B$ (min$^1$) |
| | M | 49.4±8.6 | 17.9±3.2 | 31.1±4.4 | 25.6±5.4 | 45.8±10.0 |
| 14 | F | 57.3±17.8 | 16.4±4.2 | 34.0±6.1 | 20.3±3.2 | 49.6±7.2 |
| | M | 57.2±15.2 | 17.1±3.9 | 32.1±7.9 | 23.0±3.5 | 46.1±10.6 |
| 15 | F | 67.8±15.9 | 16.6±3.3 | 31.4±3.9 | 21.1±4.1 | 47.1±5.9 |
| | M | 63.6±8.2 | 18.1±3.1 | 28.3±4.5 | 25.2±5.5 | 43.5±10.5 |
| 16 | F | 64.1±12.9 | 15.9±2.9 | 31.5±8.0 | 21.0±2.9 | 45.4±7.9 |
| | M | 69.6±10.8 | 19.5±7.7 | 26.7±7.0 | 24.5±5.6 | 40.8±6.6 |
| 17 | F | 60.7±9.3 | 15.1±2.3 | 31.2±3.7 | 22.6±5.7 | 43.9±8.3 |
| | M | 72.4±11.0 | 17.0±3.1 | 25.7±4.6 | 24.7±5.8 | 42.5±10.0 |
| 18 | F | 61.3±8.3 | 16.7±4.1 | 30.1±9.4 | 23.1±3.3 | 45.2±12.6 |
| | M | 68.7±12.1 | 15.9±2.6 | 28.8±8.6 | 23.1±5.5 | 40.3±8.1 |

The baseline calibration means that when the user starts to use a new product, the user is required to perform a baseline calibration process for more accurate monitoring results. During this process, the users input their age, body mass and gender information and wears the mask to do some moderate activities (e.g. walking at different speeds) without carrying a load.

The users' breathing tidal volume and corresponding breathing frequency data at various activity intensities are uploaded and compared with the database.

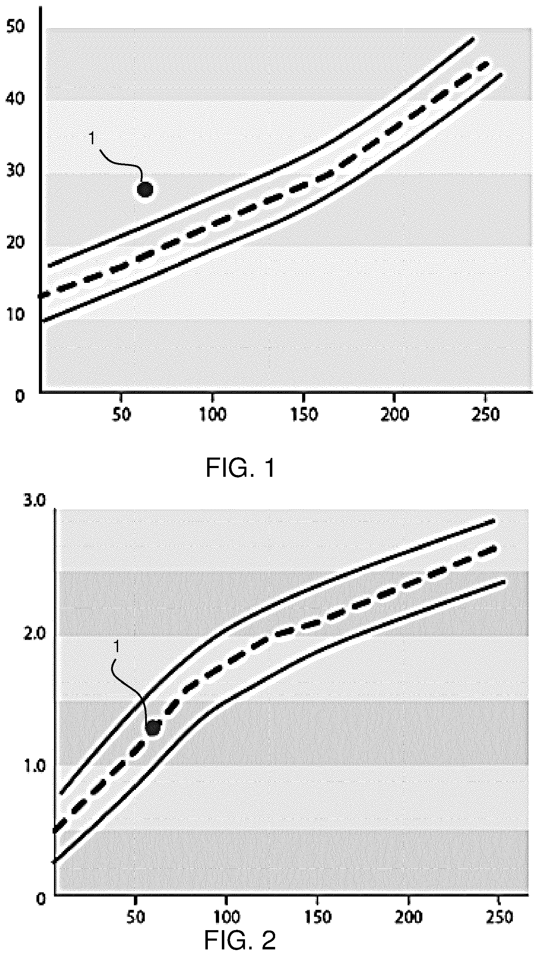

Figure 1 shows the relationship between respiratory rate (/minute, y-axis) and exercise rate (W, x-axis) and Figure 2 shows the relationship between tidal volume (L, y-axis) and exercise rate (W, x-axis).

As shown in Figures 1 and 2, for a given age, gender and body weight, there is a trend for the relationship between breathing frequency and tidal volume at various levels of activity exercise. The two solid lines in each figure respect the upper and lower boundaries of the expected relationship as stored in the database.

When the calibration baseline of the user (e.g. the dotted line Figures 1 and 2) is located within the expected boundaries for the same age, gender and similar body weight, the calibration process is passed. Of course, this calibration process will repeat during a period time of usage with the increase of user's age. During calibration stage, if the user falls outside the expected range, the system may instruct the user to do the calibration again and take care of the spinal posture during the calibration.

After calibration, the user starts to wear the mask e.g. during their daily school commute, and the breathing tidal volume and breathing frequency data are continuously monitored. Normally, with the change of activity intensity, both the breathing tidal volume and breathing frequency will change and both of them track the dotted baseline and also lie within the expected range. However, if for a given tidal volume, the corresponding breathing frequency value increases significantly compared with the expected value and is thus located outside the expected boundaries, this indicates a change of breathing pattern and signifies a poor spinal posture. An alarm signal could then be sent to user as a reminder to focus on their spinal posture.

The points 1 indicate a pair of values falling outside the expected ranges, because of a high breathing frequency. The invention thus makes use of the observation that normally both tidal volume and breathing frequency will increase with an increase of activity intensity, whereas with poor posture there is an increase in breathing frequency instead of both breathing frequency and tidal volume.

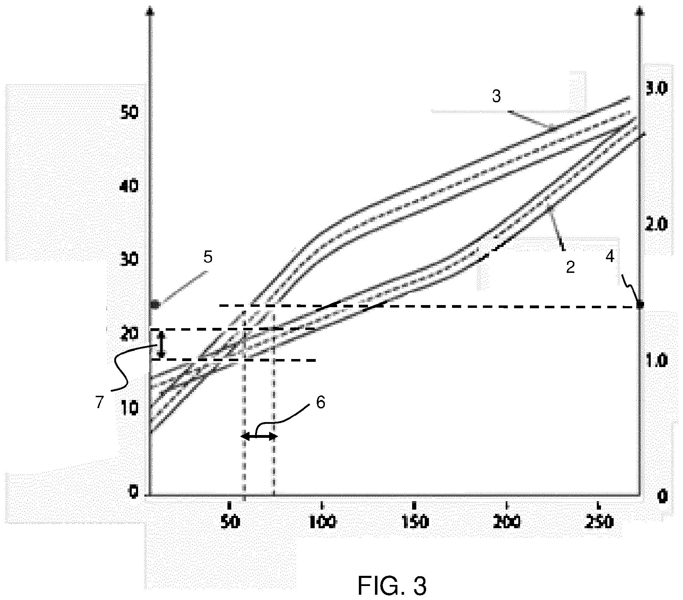

Figure 3 shows a combination of the two plots of Figures 1 and 2. The left y-axis is the respiratory rate (plot 2) and the right y-axis is the tidal volume (plot 3).

During the monitoring process after the calibration is complete, when a pair of tidal volume and breathing rate data are obtained from the fan signal (such as the two dots 4,5 in Figure 3), the corresponding normal breathing rate range can be obtained from the tested tidal volume data and the relationship built during the calibration phase.

If the measured breathing rate value is beyond the limits of the normal range, it indicates the poor posture.

For example, the measured tidal volume is shown as point 4 (with unknown activity level, hence the point is shown on the right y-axis). This corresponds to an activity range 6 between 60W and 75W. This in turn corresponds to an expected breathing rate range 7, but the measured breathing rate 5 (with unknown activity level, hence the point is shown on the left y-axis) is outside this range.

Equivalently, the measured breathing rate may be used to determine the expected range of tidal volumes, and if the measured tidal volume is outside the range, there is a departure from the pair of expected relationships.

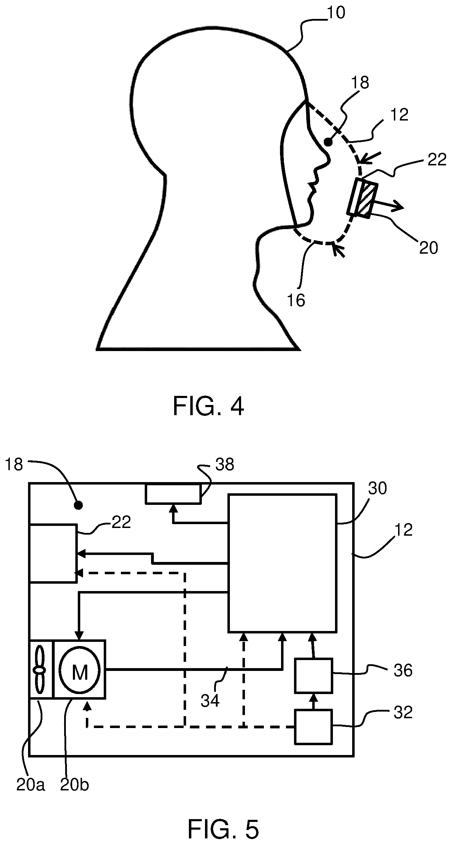

Figure 4 shows a face mask for implementing the approach explained above,

A subject 10 is shown wearing a face mask 12 which covers the nose and mouth of the subject. The primary purpose of the mask is to filter air before it is breathed in the subject. For this purpose, the mask body itself acts as an air filter 16. Air is drawn into an air chamber 18 formed by the mask by inhalation. During inhalation, an outlet valve 22 such as a check valve is closed due to the low pressure in the air chamber 18.

The filter 16 may be formed only by the body of the mask, or else there may be multiple layers. For example, the mask body may comprise an external cover formed from a porous textile material, which functions as a pre-filter. Inside the external cover, a finer filter layer is reversibly attached to the external cover. The finer filter layer may then be removed for cleaning and replacement, whereas the external cover may for example be cleaned by wiping. The external cover also performs a filtering function, for example protecting the finer filter from large debris (e.g. mud), whereas the finer filter performs the filtering of fine particulate matter. There may be more than two layers. Together, the multiple layers function as the overall filter of the mask.

When the subject breathes out, air is exhausted through the outlet valve 22. This valve is opened to enable easy exhalation, but is closed during inhalation. A fan 20 assists in the removal of air through the outlet valve 22. Preferably more air is removed than exhaled so that additional air is supplied to the face. This increases comfort due to lowering relative humidity and cooling. During inhalation, by closing the valve, it is prevented that unfiltered air is drawn in. The timing of the outlet valve 22 is thus dependent on the breathing cycle of the subject. The outlet valve may be a simple passive check valve operated by the pressure difference across the filter 16. However, it may instead be an electronically controlled valve.

There will be a varied pressure inside the chamber if the mask is worn and the user is breathing. In particular the chamber is closed by the face of the user. The pressure inside the closed chamber when the mask is worn will also vary as a function of the breathing cycle of the subject. When the subject breathes out, there will be a slight pressure increase and when the subject breathes in there will be a slight pressure reduction.

If the fan is driven with a constant drive level (i.e. voltage), the different prevailing pressure will manifest itself as a different load to the fan, since there is a different pressure drop across the fan. This altered load will then result in a different fan speed. The rotation speed of the fan may thus be used as a proxy for a measurement of pressure across the fan. This is a preferred implementation because it uses fewer sensors.

For a known pressure (e.g. atmospheric pressure) at one side of the fan, the pressure monitoring enables determination of a pressure, or at least a pressure change, on the other side of the fan. This other side is for example a closed chamber which thus has a pressure different to atmospheric pressure.

The pressure variation, as detected based on monitoring the fan rotation speed, may be used to obtain information about the breathing of the user. In particular, a first value represents the depth of breathing and a second value represents the rate of breathing.

The means for determining a rotation speed may comprise an already existing output signal from the fan motor or a separate simple sensing circuit may be provided as an additional part of the fan. However, in either case the fan itself is used so that no additional sensors are required.

Figure 5 shows one example of the components of the system. The same components as in Figure 4 are given the same reference numbers.

In addition to the components shown in Figure 4, Figure 5 shows a controller 30, a local battery 32 and a means 36 for determining the fan rotation speed. It shows an output 38 for providing output information to the user. It could be an integrated display, but more preferably it is a wireless communications transmitter (or transceiver) for sending data to a remote device such as a smartphone, which can then be used as the final user interface for providing data to the user, and optionally for receiving control commands from the user for relaying to the controller 30.

The smartphone may also be used for inputting user information to create a user profile. The user profile includes at least the age, weight and gender of the user, since these may be used to form the baseline relationships explained above.

The fan 20 comprises a fan blade 20a and a fan motor 20b. In one example, the fan motor 20b is an electronically commutated brushless motor, and the means for determining rotation speed comprises an internal sensor of the motor. Electronically commutated brushless DC fans have internal sensors that measure the position of the rotor and switch the current through the coils in such a way that the rotor rotates. The internal sensor is thus already provided in such motors to enable feedback control of the motor speed.

The motor may have an output port on which the internal sensor output 34 is provided. Thus, there is a port which carries a signal suitable for determining the rotation speed.

Alternatively, the means for determining the rotation speed may comprise a circuit 36 for detecting a ripple on the electrical supply to the motor 20b. The ripple results from switching current through the motor coils, which cause induced changes in the supply voltage as a result of the finite impedance on the battery 32. The circuit 36 for example comprises a high pass filter so that only the signals in the frequency band of the fan rotation are processed. This provides an extremely simple additional circuit, and of much lower cost than a conventional pressure sensor.

This means the motor can be of any design, including a two-wire fan with no in-built sensor output terminal. It will also work with a DC motor with brushes.

If the outlet valve 22 is an electronically switched value, the respiration cycle timing information may then be used to control the outlet valve 22 in dependence on the phase of the respiration cycle. The fan speed monitoring thus provides a simple way to determine inhalation phases, which may then be used to control the timing of the outlet valve 22 of the mask.

In addition to controlling the outlet valve, the controller may turn off the fan during an inhalation time or an exhalation time. This gives the mask different operating modes, which may be used to save power.

For a given drive level (i.e. voltage) the fan speed increases at lower pressure across the fan because of the reduced load on the fan blades. This gives rise to an increased flow. Thus, there is an inverse relationship between the fan speed and the pressure difference.

This inverse relationship may be obtained during a calibration process or it may be provided by the fan manufacturer. The calibration process for example involves analyzing the fan speed information over a period during which the subject is instructed to inhale and exhale regularly with normal breathing. The captured fan speed information can then be matched to the breathing cycle, from which threshold values can then be set for discriminating between inhalation and exhalation.

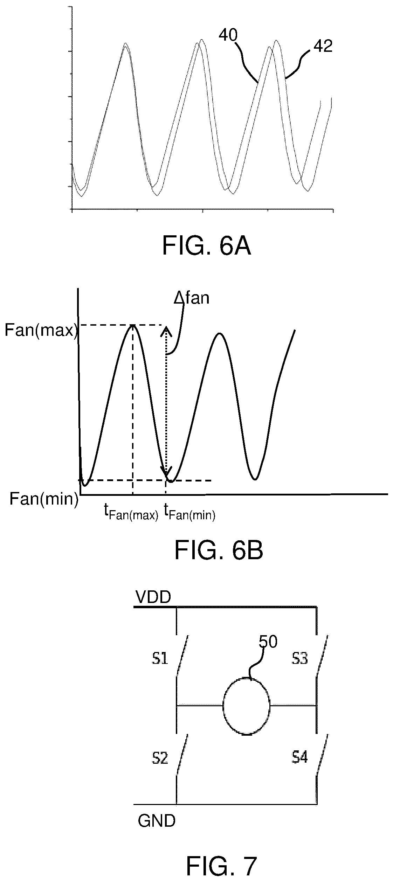

Figure 6A shows schematically the rotor position (as a measured sensor voltage) against time.

The rotational speed may be measured from the frequency of the AC component (caused by the switching events in the motor) of the DC voltage to the fan. This AC component originates from the current variation that the fan draws, imposed on the impedance of the power supply.

Figure 6A shows the signal during inhalation as plot 40 and during exhalation as plot 42. There is a frequency reduction during exhalation caused by an increased load on the fan by the increased pressure gradient. The observed frequency changes thus results from the different fan performance during the breathing cycle.

Figure 6B shows the frequency variation over time, by plotting the fan rotation speed versus time. There is a maximum difference in fan rotation speed $\Delta$fan between successive maxima and minima, and this correlates with the depth of breathing. This is the first value derived from the fan rotation signal. The time between these points is used to derive the second value, for example the frequency corresponding to this time period (which is then twice the breathing rate).

Note that the first value may be obtained from the raw fan rotation signal or there may be smoothing carried out first. Thus, there are at least two different ways to calculate the maximum swing, based on untreated real-time speeds or treated speeds. In practice, there is noise or other fluctuations added on the real-time signals. A smoothing algorithm may be used to treat the real-time signal and calculate the first value from the smoothed signal.

During the exhalation, fan operation forces air out of the area between face and mask. This enhances comfort because exhalation is made easier. It can also draw additional air onto the face which lowers the temperature and relative humidity. Between inhalation and exhalation, the fan operation increases comfort because fresh air is sucked into the space between the face and the mask thereby cooling that space.

In one example, during inhalation, the outlet valve is closed (either actively or passively) and the fan can be switched off to save power. This provides a mode of operation which is based on detecting the respiration cycle.

The precise timing of the inhalation and exhalation phases can be inferred from previous respiration cycles, if the fan is turned off for parts of the respiration cycle, and hence not giving pressure information.

For the fan assisted exhalation, power needs to be restored just before the exit valve opens again. This also makes sure that the next inhale-exhale cycle remains properly timed and sufficient pressure and flow are made available.

Around 30% power savings are easily achievable using this approach, resulting in prolonged battery life. Alternatively, the power to the fan can be increased by 30% for enhanced effectiveness.

With different fan and valve configurations the measurement of the fan rotation speed enables control to achieve increased comfort.

As mentioned above, a fan using an electronically commutated brushless DC motor has internal sensors that measure the position of the rotor and switch the current through the coils in such a way that the rotor rotates.

Figure 7 shows an H-bridge circuit which functions as an inverter to generate an alternating voltage to the stator coils 50 of the motor from a DC supply VDD, GND. The inverter has a set of switches S1 to S4 to generate an alternating voltage across the coil 50. The switches are controlled by signals which depend on the rotor position, and these rotor position signals may be used to monitor the fan rotation.

As outlined above, the fan monitoring enables the breathing rate and tidal volume to be determined. The way pressure information (or proxy pressure information) and fan rotation speed enable a breathing rate and tidal volume measurement will now be explained. An exhaling fan direction is taken as an example.

Once the intelligent mask is working, the controller can record a period of data, such as 5 seconds, which is longer than an individual breathing cycle. Within that volume of data, the maximum and minimum data point for the mask rotation speed, and the corresponding timing instants, enable the frequency to be easily calculated (see Figure 6B).

$$f = 1/2(t_{max} - t_{min}) \qquad (1)$$

For the breathing volume calculation, the inhaled or exhaled volume is to be calculated. This example is based on the exhaled breath volume. The volume depends on the breathing flow rate $FR_{nose}$ (e.g. from the nose), the fan flow rate FRfan, and the air flow through the filter. There are two scenarios:

(i) $FR_{nose} < FR_{fan}$, at this time $P_{cavity} < 0$, and the air flow direction through the filter is external to internal;
(ii) $FR_{nose} > FR_{fan}$, at this time $P_{cavity} > 0$, the air flow direction though the filter is internal to external.

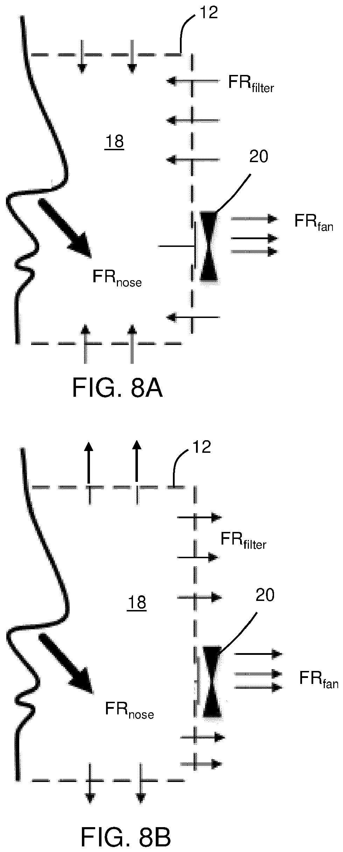

Figure 8A shows the situation with $FR_{nose} < FR_{fan}$, $P_{cavity} < 0$ with filter air flow entering the chamber 18.
Figure 8B shows a situation with $FR_{nose} > FR_{fan}$, $P_{cavity} > 0$ with the filter air flow leaving the chamber 18.
These two images both relate to exhalation, with the fan turned on.
The filter permeability performance (represented by value K) provides a linear relationship between pressure (P) and air flow through the filter $FR_{filter}$:

$$FR_{filter} = K * P \qquad (2)$$

With increasing pressure P, the flow rate (L/s) through the filter will increase. The value K is a permeability coefficient, and different filters have different value of K.
Based on a known filter permeability performance, once the pressure has been measured from the pressure sensor or indirectly measured the pressure from fan signal, the value $FR_{filter}$ is obtained.
Depending on the pressure value (or the fan signal), the flow direction through the filter is also known. As a result. The breathing flow rate $FR_{nose}$ at each moment can be calculated:

$$FR_{nose} = FR_{fan} - FR_{filter}, \ \ if \ P_{cavity} < 0 \qquad (3a)$$

This corresponds to the flows shown in Figure 8A.

$$FR_{nose} = FR_{fan} + FR_{filter}, \ \ if \ P_{cavity} > 0 \qquad (3b)$$

This corresponds to the flows shown in Figure 8B.
The fan speed (for a constant fan drive signal) satisfies the following relation:

$$n(t)/n(0) = P_{cavity}(t)/P_{cavity}(0) \qquad (4)$$

Where n(0) is the default fan speed when the cavity pressure is $P_{cavity}(0)$ where $P_{cavity}(0)$ is the baseline of the cavity pressure, which means the user is not breathing. n(t) is the fan speed at time t and when the cavity pressure is $P_{cavity}(t)$. As a result, according to the fan rotation feedback signal, it is also easy to calculate the breathing flow rate $FR_{nose}$.
According to the breathing flow rate, breath volume V can be determined as an integral over one cycle, namely one exhalation cycle in this example:

$$V = \int_{t_0}^{t_n}(FR_{nose} * t) \, dt \qquad (5)$$

Here t0 is the time at which an exhalation cycle began, $t_n$ is the time at which exhaling finished.
The invention enables posture monitoring with low cost (no additional cost for hardware, only some functions added in software), individualized measurement and high accuracy. The pressure information may be obtained from the fan speed signal, but pressure sensors may instead be used, and indeed some mask designs already include pressure sensors for breathing detection.

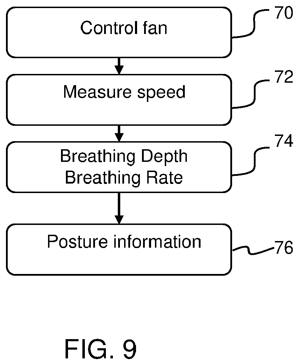

Figure 9 shows a posture monitoring method, comprising:

in step 70, controlling a fan of a mask;
in step 72, monitoring a rotation speed of the fan;
in step 74, determining from the rotation speed of the fan a first value which relates to a depth of breathing and a second value which relates to a rate of breathing; and
in step 76 determining from the first and second values information relating to the posture of the user.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A posture monitoring system, comprising:

   a mask which defines an air chamber over the nose and mouth of a user;
   a fan (20) for rotating at a rotation speed and drawing air from outside the air chamber (18) into the air chamber and/or drawing air from inside the air chamber to the outside; and
   a controller configured to:

   determine from the rotation speed of the fan a first value which relates to a depth of breathing and a second value which relates to a rate of breathing; and
   determine from the first and second values information relating to the posture of the user.

2. The system of claim 1, wherein the first value is a tidal volume.

3. The system of claim 2, further comprising a filter which forms a boundary between the air chamber and the ambient surroundings outside the air chamber, wherein the controller is configured to obtain the tidal volume by:

   determining a pressure between the air chamber and the ambient surroundings;
   analyzing the rotation speed of the fan over time and the pressure over time thereby to determine breathing flow rate information, taking into account the permeability characteristics of the filter.

4. The system of claim 3, wherein the controller is configured to derive the pressure between the air chamber and the ambient surroundings from the rotation speed of the fan, such that the fan speed is used as a proxy of pressure measurement.

5. The system of any one of claims 3 to 4, wherein the controller is configured to:

   derive a filter flow rate through the filter (16) based on the pressure and on the permeability characteristics of the filter;
   derive a fan flow rate from the fan rotation speed; and
   derive a breathing flow rate based on a sum of, or difference between, the filter flow rate and the fan flow rate.

6. The system of claim 5, wherein the controller is configured to determine the timing of inhaling and exhaling from the pressure, and to derive the breathing flow rate during inhalation or exhalation.

**7.** The system of claim 5 or 6, wherein the controller is configured to derive the tidal volume from the breathing flow rate over the time of a breathing inhalation or exhalation.

**8.** The system of any one of claims 1 to 7, wherein the first value is based on a maximum swing in fan rotation speed during a sampling window and wherein the second value is a frequency based on the time between a consecutive maxima and minima in the fan rotation speed.

**9.** The system of any one of claims 1 to 8, wherein the controller is part of a system remote to the mask, with wireless communication between the controller and the mask.

**10.** The system of any one of claims 1 to 9, wherein the controller is configured to implement a calibration routine, and thereby determine an expected relationship between each of the first and second values and an exercise intensity when the user is carrying no additional load.

**11.** The system of any one of claims 1 to 10, wherein the controller is configured to implement a monitoring function after the calibration routine, and thereby determine when the first and/or second values depart from the expected relationship with the exercise intensity, and thereby determine that the user has an incorrect posture.

**12.** A posture monitoring method, comprising:

using a controller to control a fan of a mask which defines an air chamber over the nose and mouth of a user, wherein the fan (20) is for drawing air from outside the air chamber (18) into the air chamber and/or drawing air from inside the air chamber to the outside;
monitoring a rotation speed of the fan;
determining from the rotation speed of the fan a first value which relates to a depth of breathing and a second value which relates to a rate of breathing; and
determining from the first and second values information relating to the posture of the user.

**13.** The method of claim 12, wherein determining a first value comprises determining a tidal volume, by:

determining a pressure between the air chamber and the ambient surroundings from the rotation speed of the fan, such that the fan speed is used as a proxy of pressure measurement; and
analyzing the rotation speed of the fan over time and the pressure over time thereby to determine breathing flow rate information, taking into account the permeability characteristics of the filter.

**14.** The method of claim 13, comprising:

implementing a calibration routine, and thereby determine an expected relationship between the each of the first and second values and an exercise intensity when the user is carrying no additional load; and
implementing a monitoring function after the calibration routine, and thereby determine when the first and/or second values depart from the expected relationship with the exercise intensity, and thereby determine that the user has an incorrect posture.

**15.** A computer program comprises computer program code which is adapted, when said program is run on a computer, to implement the method of any one of claims 12 to 14.

FIG. 1

FIG. 2

FIG. 3

FIG. 4

FIG. 5

FIG. 6A

FIG. 6B

FIG. 7

12

FR$_{filter}$

18

20

FR$_{fan}$

FR$_{nose}$

FIG. 8A

12

FR$_{filter}$

18

20

FR$_{fan}$

FR$_{nose}$

FIG. 8B

```
┌─────────────────────────┐
│      Control fan        │─── 70
└─────────────────────────┘
            │
            ▼
┌─────────────────────────┐
│     Measure speed       │─── 72
└─────────────────────────┘
            │
            ▼
┌─────────────────────────┐
│    Breathing Depth      │─── 74
│    Breathing Rate       │
└─────────────────────────┘
            │
            ▼
┌─────────────────────────┐
│   Posture information   │─── 76
└─────────────────────────┘
```

FIG. 9

Europäisches
Patentamt

European
Patent Office

Office européen
des brevets

## EUROPEAN SEARCH REPORT

Application Number

EP 20 18 0998

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/064520 A1 (KONINKLIJKE PHILIPS NV [NL]) 2 April 2020 (2020-04-02) * figures 1,2 * * pages 10-14 * * pages 16-17 * ----- | 1-15 | INV. A61B5/08 A61B5/09 A61B5/097 A61B5/00 |
| Y,D | JING XIAN LI ET AL: "The effect of load carriage on movement kinematics and respiratory parameters in children during walking", EUROPEAN JOURNAL OF APPLIED PHYSIOLOGY, vol. 90, no. 1-2, 29 May 2003 (2003-05-29) , pages 35-43, XP055755441, DE ISSN: 1439-6319, DOI: 10.1007/s00421-003-0848-9 * pages 35-36 * ----- | 1-15 | |
| A | EP 3 574 959 A1 (KONINKLIJKE PHILIPS NV [NL]) 4 December 2019 (2019-12-04) * paragraph [0096] * ----- | 9 | |
| A | WEBER Z J ET AL: "Resistance to nasal airflow related to changes in head posture", AMERICAN JOURNAL OF ORTHODONTICS,, vol. 80, no. 5, 1 November 1981 (1981-11-01), pages 536-545, XP023063760, ISSN: 0002-9416, DOI: 10.1016/0002-9416(81)90248-7 [retrieved on 1981-11-01] * the whole document * ----- | 1-15 | TECHNICAL FIELDS SEARCHED (IPC) A61B A62B A61M |

The present search report has been drawn up for all claims

| Place of search | Date of completion of the search | Examiner |
|---|---|---|
| The Hague | 1 December 2020 | Vanderperren, Yves |

EPO FORM 1503 03.82 (P04C01)

## ANNEX TO THE EUROPEAN SEARCH REPORT
## ON EUROPEAN PATENT APPLICATION NO.

EP 20 18 0998

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2020

| Patent document cited in search report | | | Publication date | Patent family member(s) | | | Publication date |
|---|---|---|---|---|---|---|---|
| WO 2020064520 | A1 | | 02-04-2020 | CN | 110947114 | A | 03-04-2020 |
| | | | | CN | 211751892 | U | 27-10-2020 |
| | | | | WO | 2020064520 | A1 | 02-04-2020 |
| EP 3574959 | A1 | | 04-12-2019 | NONE | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018215225 A

**Non-patent literature cited in the description**

- **JING XIAN LI ; YOULIAN HONG ; PAUL D. ROBINSON.** The effect of load carriage of movement kinematics and respiratory parameters in children during walking. *Eur J Appl Physiol,* 2003, vol. 90, 35-43

- **KRISTIN S. ONDRAK ; ROBERT G. MCMURRAY.** Exercise-induced breathing patterns of youth are related to age and intensity. *Eur J Appl Physiol,* 2006, vol. 98, 88-96